(19) Europäisches Patentamt
European Patent Office
Office européen des brevets

(11) Veröffentlichungsnummer: **0 380 783**
**A2**

(12) **EUROPÄISCHE PATENTANMELDUNG**

(21) Anmeldenummer: **89122424.8**

(22) Anmeldetag: **05.12.89**

(51) Int. Cl.5: **C07C 49/16, C07C 45/63, C07C 45/59**

(30) Priorität: **02.02.89 DE 3903029**

(43) Veröffentlichungstag der Anmeldung: **08.08.90 Patentblatt 90/32**

(84) Benannte Vertragsstaaten: **AT BE CH DE FR GB IT LI NL SE**

(71) Anmelder: **HÜLS AKTIENGESELLSCHAFT**
**Patentabteilung / PB 15 - Postfach 13 20**
**D-4370 Marl 1(DE)**

(72) Erfinder: **Kaufhold, Manfred, Dr.**
**Jasminweg 20**
**D-4370 Marl(DE)**
Erfinder: **Otte, Werner, Dr.**
**Eichenstück 55**
**D-4270 Dorsten 11(DE)**

(54) **Verfahren zur Herstellung von 5-Chlorpentanon-2.**

(57) Es war Aufgabe der Erfindung, ein einfaches und umweltfreundliches Verfahren zur Herstellung von 5-Chlorpentanon-2, ausgehend von einem Ketal eines Lävulinsäureesters, dessen Hydrierung und anschließende Umsetzung mit Salzsäure, zu entwickeln.

Die Lösung besteht darin, daß man zunächst das Ketal des Lävulinsäureesters destillativ reinigt, die Hydrierungskatalysatoren für das Ketal alkalisch einstellt und das bei dieser Hydrierung entstehende 3-(2-Methyl-1,3-dioxolan-2-yl)-propan-1-ol mit Salzsäure zum 5-Chlorpentanon-2 umgesetzt wird.

Verwendung als Pharma-Vorprodukt sowie zur Herstellung von Cyclopropylmethylketon.

EP 0 380 783 A2

## Verfahren zur Herstellung von 5-Chlorpentanon-2

Die Erfindung betrifft ein Verfahren zur Herstellung von 5-Chlor-pentanon-2 durch Ketalisierung eines Lävulinsäureesters mit Ethylenglykol zu (1), katalytische Hydrierung von (1) zu 3-(2-Methyl-1,3-dioxolan-2-yl)-propan-1-ol (2) und Umsetzung dieses Dioxolanderivats mit Salzsäure zum 5-Chlorpentanon-2 (3).

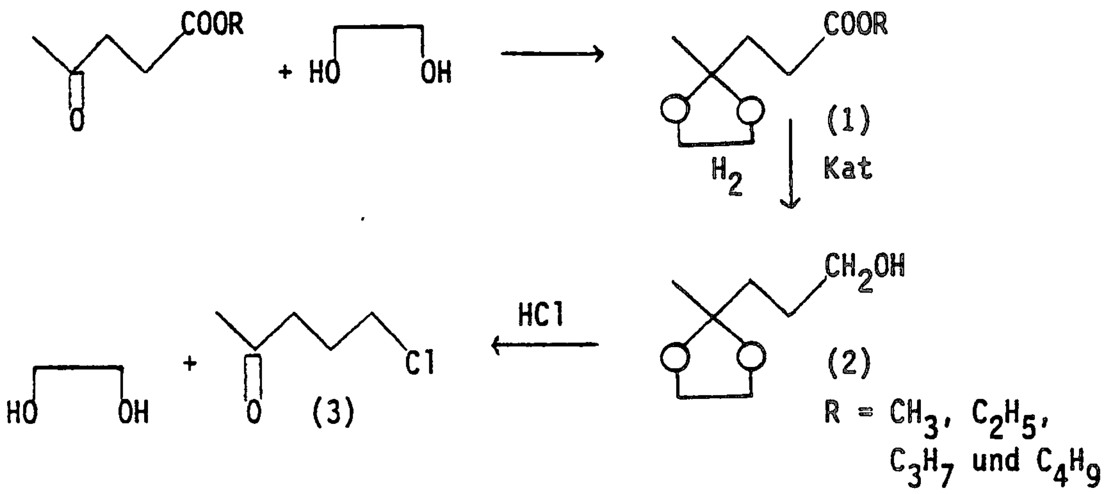

(1)
(2)
(3)
R = $CH_3$, $C_2H_5$, $C_3H_7$ und $C_4H_9$

Synthesen von 5-Chlorpentanon sind aus der Literatur seit langem bekannt:
Die gängigste Methode ist die Umsetzung von alpha-Acetyl-gamma-butyrolacton mit Chlorwasserstoff (Org. Synth. CV4, 1963, 597). Nachteilig bei diesem Verfahren ist der Einsatz des kostspieligen alpha-Acetyl-gamma-butyrolactons, das durch Esterkondensation von z. B. gamma-Butyrolacton und Ethylacetat in Gegenwart von äquimolaren Mengen an metallischem Natrium zugänglich ist.

Zahlreiche Autoren beschreiben die Umsetzung von 5-Hydroxypentanon-2 mit Chlorwasserstoff zu (3), z. B. Ishimaru, C.A. 1957, 17728. Auch 5-Hydroxypentanon-2 ist eine kostspielige Chemikalie, die das Verfahren unwirtschaftlich macht.

Dagegen setzen L. Willimann und H. Schinz, s. Helvetia Chem. Akta, 32, 1949, 2151, den preiswerten Lävulinsäureethylester ein ($R = C_2H_5$), ketalisieren ihn ebenfalls mit Ethylenglykol und reduzieren (1) mit einem großen Überschuß an metallischem Natrium und Ethanol zu (2). Der generell hohe herstellungsbedingte Chlorgehalt des Lävulinsäureesters stört bei dieser Hydrierart des Ketals nicht. Nachteilig bei diesem Verfahren ist die Verwendung von kostspieligem und nur mit Vorsicht zu handhabendem metallischem Natrium sowie die Beseitigung der dadurch entstehenden Abfallprodukte.

Alle bekannten Verfahren zur Herstellung von 5-Chlorpentanon-2 gehen entweder von kostspieligen Einsatzstoffen aus oder verwenden im Verlauf der Synthese teure Chemikalien in großen Mengen, deren Vernichtung Umweltschutzprobleme mit sich bringen. Wünschenswert wäre ein Verfahren, mit dem man das Ketal eines Lävulinsäureesters (1) unter üblichen Bedingungen katalytisch zu (2) hydrieren könnte.
Die Umsetzung von (2) mit Salzsäure zu (3) sollte problemlos sein, da man nach Willimann und Schinz aus (2) durch Reaktion mit Wasser und wenig Säure 5-Hydroxypentanon-2 herstellen kann, dessen Umsetzung mit Salzsäure zum 5-Chlorpentanon-2 (3), wie oben erwähnt, ebenfalls in der Literatur beschrieben ist.

Es bestand daher die Aufgabe, ein Verfahren zu entwickeln, nach dem man bei geringem technischen Aufwand und ohne den Einsatz von teuren Reagenzien 3-(2-Methyl-1,3-dioxolan-2-yl)-propan-1-ol) (2) herstellen kann, aus dem man durch Umsetzung mit Salzsäure das 5-Chlorpentanon-2 gewinnt, das große wirtschaftliche Bedeutung als Pharma-Vorprodukt und als Ausgangsprodukt zur Herstellung von Cyclopropylmethylketon hat.

Wenn man das Ketal eines Lävulinsäureesters (1) katalytisch so hydriert, wie man üblicherweise Esterhydrierungen durchführt, z. B. mit einem handelsüblichen Kupferchromitkatalysator, erhält man Pentandiol-1,4, d. h. der Dioxolan-Ring wird hydrierend gespalten. Überraschenderweise wurde gefunden, daß die Hydrierung unter sonst gleichen Bedingungen in die gewünschte Richtung zum 3-(2-Methyl-1,3-dioxolan-2-yl)-propan-1-ol (2) geht, wenn man den Katalysator alkalisch einstellt.

Gegenstand der Erfindung ist ein Verfahren zur Herstellung von 5-Chlorpentanon-2, ausgehend von Lävulinsäureester, welches gekennzeichnet ist durch

a) Ketalisierung des Lävulinsäureesters $CH_3$-CO-$CH_2$-$CH_2$-COOR, worin R = $CH_3$, $C_2H_5$, $C_3H_7$ und $C_4H_9$ mit Ethylenglykol in an sich bekanner Weise

b) destillative Reinigung des Ketals auf einen Chlorgehalt ≤ 10 ppm

c) Hydrierung des gereinigten Ketals mit einem an sich üblichen Hydrierkatalysator, der vorher mit einer alkoholischen Alkali-und/oder Erdalkalihydroxidlösung behandelt wurde, zu 3-(2-Methyl-1,3-dioxalon-2-yl-propan-1-ol) und

d) Umsetzung des 3-(2-Methyl-1,3-dioxalon-2-yl-propan-1-ol) mit Salzsäure zu 5-Chlorpentanon-2.

Die Normalität der alkoholischen Alkali- und/oder Erdalkalilösung beträgt 0,001 bis 0,5, vorzugsweise 0,005 bis 0,1 und besonders bevorzugt 0,05.

Geeignete Alkali- und Erdalkaliverbindungen sind LiOH, NaOH, KOH, $Mg(OH)_2$, $Ca(OH)_2$, $Sr(OH)_2$, $Ba(OH)_2$. Besonders geeignet sind NaOH, KOH und $Ba(OH)_2$.

Die Herstellung der Ausgangsverbindung (1) erfolgt beispielsweise durch Umsetzung von Ethylenglykol mit Lävulinsäurebutylester in Gegenwart von Phosphorsäure als Katalysator und Cyclohexan als Schleppmittel zum Wasserauskreisen. Nach destillativer Aufarbeitung erhält man reines Ketal des Lävulinsäurebutylesters mit niedrigem Chlorgehalt von = 10 ppm. Dieser Effekt ist überraschend, da der Chlorgehalt des eingesetzten Butylesters herstellungsbedingt stets hoch liegt, z. B. bei 67 ppm. Produkte mit derartig hohen (> 10 ppm) Chlorgehalten können mit üblichen Hydrierkatalysatoren nicht hydriert werden, da durch den Chlorgehalt bedingt der Katalysator schnell an Aktivität verlieren würde und mit Korrosion bei den Hochdruckreaktoren zu rechnen ist.

Die anschließende Hydrierung erfolgt mit üblichen Hydrierkatalysatoren, wie Kupferchromitkatalysatoren (z. B. Mallinckrodt E 406, Harshaw 1107, Girdler G 99), die erfindungsgemäß behandelt werden.

Die gaschromatographische Analyse des Hydrieraustrags zeigt einen überraschend hohen Umsatz von über 99 % und eine Selektivität von über 95 %. Die Aufarbeitung des Hydrieraustrages erfolgt wie üblich durch Destillation.

Die Herstellung von 5-Chlorpentanon-2 aus (2) erfolgt in analoger Weise wie in der Literatur beim 5-Hydroxipentanon-2 beschrieben:
Konzentrierte Salzsäure wird vorgelegt, auf ca. 0 °C abgekühlt und bei dieser Temperatur wird (2) zugetropft. Danach wird das Gemisch andestilliert - wobei ein zweiphasiges Gemisch von 5-Chlorpentanon-2 und Wasser anfällt - und zwar so lange, bis der Destillatanfall einphasig ist, d. h. nur Wasser überdestilliert.

Das überdestillierte Gemisch wird durch Extraktion und anschließende Destillation aufgearbeitet.

Die folgenden Beispiele sollen das erfindungsgemäße Verfahren näher erläutern.

Beispiel 1 a Ketalisierung des Lävulinsäureesters und destillative Reinigung

Man benutzt eine Glasapparatur, die aus einem Dreihalskolben mit Rührer, Thermometer und Wasserauskreiser mit Rückflußkühler besteht.

Man setzt ein:
1 054 g (= 6 Mol) Lävulinsäure-n-butylester (98,1 %ig Chlorgehalt 67 ppm)
435 g (= 7 Mol) Ethylenglykol
12 g Phosphorsäure (85 %ig)
600 g Cyclohexan als Löse- und Schleppmittel

Unter Rühren wird das Gemisch zum Sieden erhitzt, wobei sich eine Temperatur von 100 °C einstellt und das anfallende Wasser ausgekreist wird. Wenn die Temperatur ansteigt, z. B. nach ca. 5,5 Stunden, wird über den Tropftrichter 200 g Cyclohexan nachgegeben. Nach 6,5 Stunden werden 2,5 ml Phosphorsäure (85 %ig) zugegeben und nach 13,5 Stunden wird das Wasserauskreisen beendet. Zum Rohprodukt werden nach dem Abkühlen auf Raumtemperatur 500 g 10 %ige Natronlauge unter Rühren gegeben und dann die Phasen getrennt:
Ölphase : 1 652 g
wäßrige Phase : 692 g

Zur Ölphase werden 3 g Soda zugegeben und an einer 0,5 m langen mit Multifil-Füllkörpern gefüllten Kolonne destilliert. Einsatz: 1 652 g

| Fr. Nr. | Temperaturen °C | | Gewicht g | Druck hPa | Verhältnis Rücklauf zur Abnahme |
|---|---|---|---|---|---|
| | Kopf | Sumpf | | | |
| 1 | 40- 58 | 56-124 | 446 | 300 | 1 : 1 |
| 2 | 25-101 | 104-162 | 27 | 133 | 1 : 1 |
| | | | | 30 | |
| 3 | 127-130 | 158-160 | 57 | 30 | 1 : 1 |
| | | | | | 3 : 1 |
| 4 | 140-148 | 160 | 12 | 30 | 3 : 1 |
| 5 | 150-152 | 156-162 | 993 | 30 | 1 : 1 |
| | | | | | 3 : 1 |
| Rückstand | | | | 23 | |
| Kühlfalle | | | | 80 | |
| | | | | 1 638 | |

Die Fraktion 1 ist Cyclohexan und wird verworfen. Die Fraktion 2 besteht zu 99 % aus n-Butanol und die Fraktion 3 zu 93 % aus nicht umgesetztem Lävulinsäure-n-butylester.
Fraktion 4 ist ein Zwischenlauf mit 75 % Lävulinsäureester und 22 % Ketal dieses Esters.
Der Hauptlauf enthält das gewünschte Ketal mit einer Reinheit von 99,6 %. Sein niedriger Chlorgehalt von 6 ppm ermöglicht die katalytische Hydrierung. Aus diesen Zahlen errechnet sich eine Ausbeute an Ketalester von ca. 77 %, bezogen auf Einsatz.

Beispiel 1 b Hydrierung des gereinigten Ketals

In einem Durchflußhydrierreaktor mit 400 ml Volumen werden stündlich 100 ml des destillativ gereinigten Ketals des Lävulinsäureesters (1) bei 300 hPa Gesamtdruck und 200 °C an einem handelsüblichen Kupferchromitkatalysator der Firma Mallinckrodt hydriert. Der Katalysator wurde vor der Hydrierung mit 2 l 0,05 n alkoholischer KOH behandelt.

Der Hydrieraustrag zeigte nach gaschromatographischer Analyse die Gehalte von folgenden Komponenten:
Butanol : 44 %
(2) : 52 %
(1) : 0,3 %

Die destillative Aufarbeitung lieferte 3-(2-Methyl-1,3-dioxalon-2-yl)-propan-1-ol (2) bei einem Siedepunkt von 127 °C bei 30 mbar in 99 %iger Reinheit.

Der Umsatz bei der Hydrierung lag also über 99 % und die Selektivität über 95 %.

Beispiel 1 c Umsetzung des hydrierten Ketals mit Salzsäure

Man benutzt eine Glasapparatur, die aus einem Dreihalskolben mit Rührer, Thermometer, Tropftrichter und Destillationsapparatur besteht.

Man setzt ein:
750 ml konzentrierte Salzsäure
292,4 g ( = 2 Mol) 3-(2-Methyl-1,3-dioxolan-2-yl)-propan-1-ol (2)

Die konzentrierte Salzsäure wird vorgelegt und auf 0 °C gekühlt. Unter Rühren wird (2) innerhalb einer halben Stunde zugetropft, wobei durch starkes Kühlen die Temperatur bei 0 °C gehalten wird. Danach wird die Kühlung entfernt und innerhalb einer halben Stunde die Temperatur soweit erhöht, das Produkt abdestilliert. Das Destillat fällt zunächst einpha sig und dann zweiphasig an. Wenn keine zweite Phase mehr anfällt, ist die Destillation beendet. Danach werden die Phasen getrennt, die wäßrige Phase mit Cyclohexan extrahiert und der Extrakt und die organische Phase zusammengegeben und destilliert. Siedepunkt des 5-Chlorpentanons bei 30 hPa = 77 °C, Ausbeute = 180 g, Reinheit = 98 %, d. h. Ausbeute auf Einsatz bezogen = 73 % d. Th.

Beispiel 1 d

Man benutzt die im Beispiel 1 c beschriebene Apparatur und führt die hier beschriebene Umsetzung bei 0 °C durch. Nachdem die Kühlung entfernt wird, gibt man 200 g Toluol hinzu und erwärmt dann bis zum Sieden am Rückfluß.

Nach einer halben Stunde Sieden wird abgekühlt, die Phasen werden getrennt und die wäßrige Phase dreimal mit Toluol extrahiert. Die Toluolphasen werden zusammengegeben und destillativ aufgearbeitet. Ausbeute, bezogen auf Einsatz: 92 % d. Th.

Beispiel 2 b

Ausführung wie Beispiel 1 b, jedoch wird der Katalysator vor der Hydrierung mit 10 l 0,01 n alkoholischer KOH-Lösung behandelt. Das Hydrierergebnis entspricht dem von Beispiel 1 b.

Beispiel 3 b

Ausführung wie Beispiel 1 b, jedoch wird der Katalysator vor der Hydrierung mit 40 ml 0,25 n alkoholischer NaOH-Lösung behandelt. Das Hydrierergebnis entspricht dem von Beispiel 1 b.

Beispiel 4 b

Ausführung wie Beispiel 1 b, jedoch wird der Katalysator vor der Hydrierung mit 2 l 0,05 n alkoholischer $Ba(OH)_2$-Lösung behandelt. Das Hydrierergebnis entspricht dem von Beispiel 1 b.

Beispiel 4 Vergleich

Die Hydrierung wurde wie in Beispiel 1 b ausgeführt, jedoch war der Katalysator nicht alkalisch eingestellt.

Der Hydrieraustrag zeigte nach gaschromatographischer Analyse folgende Zusammensetzung (Hauptkomponenten):

| | |
|---|---|
| Butanol | 51 % |
| Pentandiol-1,4 | 46 % |
| 3-(2-Methyl-1,3-dioxolan-2-yl)-propan-1-ol | 0,5 % |

## Ansprüche

1. Verfahren zur Herstellung von 5-Chlorpentanon-2, ausgehend von Lävulinsäureester, gekennzeichnet durch

a) Ketalisierung des Lävulinsäureesters $CH_3$-CO-$CH_2$-$CH_2$-COOR, worin R = $CH_3$, $C_2H_5$, $C_3H_7$ oder $C_4H_9$ mit Ethylenglykol in an sich bekannter Weise

b) destillative Reinigung des Ketals auf einen Chlorgehalt ≤ 10 ppm

c) Hydrierung des gereinigten Ketals mit einem an sich üblichen Hydrierkatalysator, der vorher mit einer alkoholischen Alkali-und/oder Erdalkalihydroxidlösung behandelt wurde, zu 3-(2-Methyl-1,3-dioxolan-2-yl-propan-1-ol) und

d) Umsetzung des 3-(2-Methyl-1,3-dioxolan-2-yl-propan-1-ol) mit Salzsäure zu 5-Chlorpentanon-2.

2. Verfahren nach Anspruch 1, dadurch gekennzeichnet, daß in der Hydrierstufe c der Hydrierkatalysator mit einer alkoholischen Alkali- und/oder Erdalkalihydroxidlösung behandelt wird, deren Normalität 0,001 bis 0,5 beträgt.

3. Verfahren nach Anspruch 2, dadurch gekennzeichnet, daß die Normalität der alkoholischen Alkali- und/oder Erdalkalihydroxidlösung vorzugsweise 0,005 bis 0,1 beträgt.

4. Verfahren nach Anspruch 2, dadurch gekennzeichnet, daß die Normalität der alkoholischen Alkali- und/oder Erdalkalihydroxidlösung besonders bevorzugt 0,05 beträgt.